(19) Europäisches Patentamt
European Patent Office
Office européen des brevets

(11) Numéro de publication: **0 022 705**
**B1**

(12) # FASCICULE DE BREVET EUROPEEN

(45) Date de publication du fascicule du brevet: **06.04.83**

(51) Int. Cl.³: **C 07 D 401/04**, A 61 K 31/44 // C07D209/08, C07D405/14

(21) Numéro de dépôt: **80401005.6**

(22) Date de dépôt: **02.07.80**

(54) **Nouveaux dérivés du tétrahydropyridinyl-indole et leurs sels, leur préparation, leur application comme médicaments et les compositions les renfermant.**

(30) Priorité: **13.07.79 FR 7918217**

(43) Date de publication de la demande: **21.01.81 Bulletin 81/3**

(45) Mention de la délivrance du brevet: **06.04.83 Bulletin 83/14**

(84) Etats contractants désignés: **AT BE CH DE FR GB IT LI LU NL SE**

(56) Documents cités:
**SU-A-639 243**

**Journal of organic chemistry, vol. 40, n° 17, 22 août 1975 K. Freter «3-cycloalkenylindoles», pages 2525-2529**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Guillaume, Jacques, 11, Allée Hélène Boucher, F-93270 Sevran (FR)**
Inventeur: **Nedelec, Lucien, 45,Bd.de L'Ouest, F-93340 Le Raincy (FR)**
Inventeur: **Dumont, Claude, 33, rue du Maréchal Vaillant, F-94130 Nogent sur Marne (FR)**

(74) Mandataire: **Vieillefosse, Jean-Claude et al, ROUSSEL-UCLAF Boîte postale no. 9 102, route de Noisy, F-93230 Romainville (FR)**

**EP 0 022 705 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés du tétrahydropyridinylindole et leurs sels, leur préparation, leur application comme médicaments et les compositions les renfermant.

La présente invention concerne de nouveaux dérivés N-substitués du tétrahydropyridinylindole ainsi que leurs sels, le procédé de préparation, l'application à titre de médicaments de ces nouveaux dérivés et les compositions les renfermant.

L'article de «Pharma Research Canada Ltd» paru dans le Journal of organic chemistry, vol. 40, nº 17, p. 2525-2529 avait déjà écrit d'un point de vue chimique un certain nombre de dérivés du tétrahydropyridinylindole.

L'invention a pour objet de nouveaux dérivés N-substitués du tétrahydropyridinylindole, ainsi que leurs sels d'addition avec les acides minéraux ou organiques, caractérisés en ce qu'ils répondent à la formule générale I:

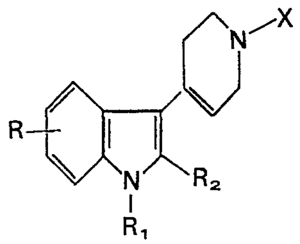

(I)

dans laquelle X représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone, hydroxyalcoyle dans lequel le radical alcoyle renferme de 2 à 5 atomes de carbone ou phénoxyalcoyle dans lequel le radical alcoyle renferme de 1 à 5 atomes de carbone, R représente un atome d'hydrogène ou d'halogène ou un radical alcoxy renfermant de 1 à 3 atomes de carbone, nitro, amino, trifluorométhyle ou méthylthio, $R_1$ et $R_2$ représentent un atome d'hydrogène ou un radical alcoyle renfermant de 1 à 3 atomes de carbone, étant entendu que si R représente un atome d'hydrogène, d'halogène ou un radical alcoxy renfermant de 1 à 3 atomes de carbone, X représente obligatoirement un radical hydroxyalcoyle ou phénoxyalcoyle tels que définis ci-dessus. Les dérivés de formule I objet du disclaimer ci-dessus font l'objet de la demande de brevet européen nº 79400019 (nº de publ. 3199).

Dans la formule générale I et dans ce qui suit, le terme radical alcoyle renfermant de 1 à 6 atomes de carbone désigne, par exemple, un radical méthyl, éthyl, propyl, isopropyl, butyl, isobutyl, pentyl ou isopentyl, le terme cycloalcoyle renfermant 5 ou 6 atomes de carbone désigne un radical cyclopentyl ou cyclohexyl, le terme radical cycloalcoylalcoyle renfermant de 4 à 7 atomes de carbone désigne, par exemple, un radical cyclopropylméthyl, le terme radical alcényle renfermant de 2 à 5 atomes de carbone, désigne par exemple, un radical vinyl, allyl, butène 2-yl ou pentène 2-yl, le terme radical alcynyle renfermant de 3 à 5 atomes de carbone, désigne, par exemple, un radical propargyl, le terme radical aralcoyle peut désigner, par exemple, un radical benzyl ou phénéthyl, le terme radical hydroxyalcoyle dans lequel le radical alcoyle renferme de 2 à 5 atomes de carbone désigne, par exemple, un radical hydroxyéthyl, hydroxypropyl, hydroxybutyl ou hydroxypentyl, le terme radical phénoxyalcoyle dans lequel le radical alcoyle renferme de 1 à 5 atomes de carbone désigne, par exemple, un radical phénoxyméthyl, phénoxyéthyl, phénoxypropyl, phénoxybutyl ou phénoxypentyl, l'atome d'halogène peut être un atome de fluor, de chlore ou de brome, le terme radical alcoxy renfermant de 1 à 3 atomes de carbone désigne, par exemple, un radical méthoxy, éthoxy ou propoxy, le terme radical alcoyle renfermant de 1 à 3 atomes de carbone désigne, par exemple, un radical méthyl, éthyl ou propyl.

Le substituant R peut être en toutes positions sur l'indole, mais de préférence en position 5 ou 6 et plus particulièrement en position 5.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits objet de l'invention, on peut citer notamment les dérivés répondant à la formule I ci-dessus, ainsi que leurs sels d'addition avec les acides minéraux ou organiques, caractérisés en ce que, dans ladite formule I, X représente un radical hydroxyalcoyle dans lequel le radical alcoyle renferme de 2 à 5 atomes de carbone et R, $R_1$ et $R_2$ ont la signification déjà indiquée. On peut citer également les dérivés répondant à la formule I ci-dessus, caractérisés en ce que X représente un radical phénoxyalcoyle dans lequel le radical alcoyle renferme de 1 à 5 atomes de carbone et R, $R_1$ et $R_2$ ont la signification déjà indiquée, de même que ceux caractérisés en ce que X représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone et $R_1$ et $R_2$ ont la signification déjà indiquée, ainsi que leurs sels d'addition avec les acides minéraux ou organiques.

Parmi les dérivés, objet de l'invention, on peut citer plus particulièrement les dérivés décrits dans les exemples et tout particulièrement le chlorhydrate de 5-nitro 3-(1-propyl-1,2,3,6-tétrahydropyridin-4-yl)-1H-indole.

L'invention a aussi pour objet un procédé de préparation des dérivés, tels que définis par la formule I ci-dessus, ainsi que de leurs sels dans laquelle X représente un radical hydroxyalcoyle dans lequel le radical alcoyle renferme de 2 à 5 atomes de carbone et R, $R_1$ et $R_2$ ont la signification déjà indiquée, caractérisée en ce que l'on fait réagir un produit de formule:

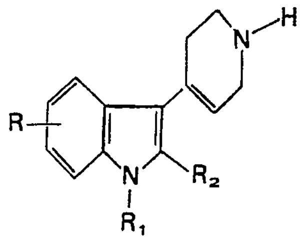

(II)

dans laquelle R, $R_1$ et $R_2$ ont la signification déjà énoncée, avec un halogénure de formule:

Hal–X' (III)

dans laquelle Hal représente un atome de chlore, de brome ou d'iode et X' représente un radical tétrahydropyranyloxy alcoyle de formule:

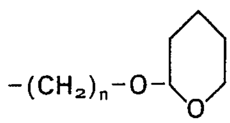

(IV)

dans laquelle n représente un nombre entier égal à 2,3,4 ou 5, pour obtenir un dérivé de formule:

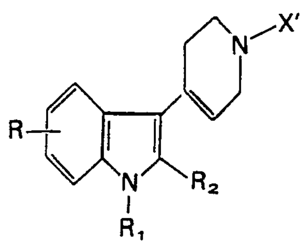

(V)

dans laquelle X', R, $R_1$ et $R_2$ ont la signification déjà indiquée, produit de formule V que l'on hydrolyse pour obtenir un produit de formule I dans laquelle X représente un radical hydroxyalcoyle dans lequel le radical alcoyle renferme de 2 à 5 atomes de carbone et R, $R_1$ et $R_2$ ont la signification déjà indiquée, que l'on isole, et, le cas échéant, traite par un acide pour en former le sel.

Dans des conditions préférentielles de mise en œuvre de l'invention, le procédé de préparation des dérivés de formule I ci-dessus décrit est caractérisé en ce que:

– la réaction du produit de formule II avec l'halogénure de formule III est effectuée au sein d'un solvant organique tel que l'isobutylméthylcétone en présence de carbonate de sodium;

– l'hydrolyse du produit de formule V est effectuée à l'aide d'une solution d'acide chlorhydrique dans un alcanol tel que le méthanol ou l'éthanol.

L'invention a également pour objet un procédé de préparation des dérivés tels que définis par la formule I ainsi que de leurs sels dans laquelle X représente un radical phénoxyalcoyle dans lequel le radical alcoyle renferme de 1 à 5 atomes de carbone, un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone et R, $R_1$ et $R_2$ ont la signification déjà énoncée, caractérisé en ce que l'on fait réagir un produit de formule:

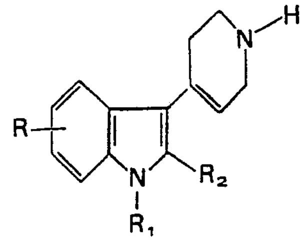

(II)

dans laquelle R, $R_1$ et $R_2$ ont la signification déjà indiquée, avec un halogénure de formule générale:

Hal–X'' (III')

dans laquelle Hal représente un atome de chlore, de brome ou d'iode et X'' représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone ou phénoxyalcoyle dans lequel le radical alcoyle renferme de 1 à 5 atomes de carbone, pour obtenir un produit de formule I dans laquelle R, $R_1$, $R_2$ et X ont la signification déjà indiquée, que l'on isole et, le cas échéant, traite par un acide pour en former le sel.

Dans des conditions préférentielles de mise en œuvre de l'invention, le procédé de préparation ci-dessus décrit est caractérisé en ce que:

– la réaction du produit de formule II avec l'halogénure de formule III' est effectuée au sein d'un solvant organique tel que le diméthylformamide en présence de carbonate de sodium, la réaction peut aussi être menée dans l'acétone ou l'isobutylméthylcétone en présence d'oxyde d'argent, de carbonate de sodium ou de triéthylamine.

Les dérivés de formule I présentent un caractère basique. On peut avantageusement préparer les sels d'addition avec les acides des dérivés de formule I en faisant réagir, en proportions sensiblement stœchiométriques, un acide minéral ou organique avec ledit dérivé de formule I. Les sels peuvent être préparés sans isoler les bases correspondantes.

Les dérivés, objet de la présente invention, possèdent de très intéressantes propriétés pharmacologiques; ils sont doués, notamment, de remarquables propriétés neuroleptiques, antipsychotiques et antiémétiques.

Ces propriétés sont illustrées plus loin dans la partie expérimentale.

Ces propriétés justifient l'utilisation des dérivés N-substitués du tétrahydropyridinylindole de formule I ainsi que de leurs sels pharmaceutiquement acceptables, à titre de médicaments.

La présente demande a ainsi également pour objet l'application, à titre de médicaments, des dérivés N-substitués du tétrahydropyridinylindole, tels que définis par la formule I ainsi que de leurs sels d'addition avec les acides pharmaceutiquement acceptables.

Parmi les médicaments objet de l'invention, on peut citer les médicaments caractérisés en ce qu'ils sont constitués par les nouveaux dérivés N-substitués du tétrahydropyridinylindole répondant à la formule I, dans laquelle X représente un radical hydroxyalcoyle dans lequel le radical alcoyle renferme de 2 à 5 atomes de carbone et R, $R_1$ et $R_2$ ont la signification déjà indiquée ainsi que par leurs sels d'addition avec les acides pharmaceutiquement acceptables, par ceux répondant à la formule I dans laquelle X représente un radical phénoxyalcoyle dans lequel le radical alcoyle renferme de 1 à 5 atomes de carbone et R, $R_1$ et $R_2$ ont la signification déjà indiquée, ainsi que par leurs sels d'addition avec les acides pharmaceutiquement acceptables et par ceux répondant à la formule I dans laquelle X représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone, R représente un radical nitro, amino, trifluorométhyl ou méthylthio et $R_1$ et $R_2$ ont la signification déjà indiquée ainsi que par leurs sels d'addition avec les acides pharmaceutiquement acceptables.

Parmi les médicaments objet de l'invention, on retient tout particulièrement le dérivé dont le nom suit:

– le chlorhydrate de 5-nitro 3-(1-propyl 1,2,3,6-tétrahydropyridin-4-yl)-1H-indole.

Les médicaments selon l'invention trouvent leur emploi par exemple dans le traitement des troubles psychiques, des troubles du comportement, des troubles caractériels ainsi que dans le traitement des vomissements et nausées de toutes origines.

La dose usuelle, variable selon le dérivé utilisé, le sujet traité et l'affection en cause, peut être par exemple de 5 mg à 200 mg par jour, par voie orale, chez l'homme.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables, peuvent être incorporés dans des compositions pharmaceutiques destinées à la voie digestive ou parentérale.

Ces compositions pharmaceutiques peuvent être par exemple solides ou liquides et se présenter sous les formes pharmaceutiques couramment utilisées en médecine humaine, comme par exemple les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule II lorsqu'ils ne sont pas connus peuvent être préparés par réaction d'un produit de formule VI:

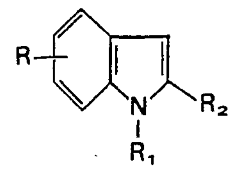

(VI)

dans laquelle R, $R_1$ et $R_2$ ont la signification déjà indiquée, avec du chlorhydrate de 4-pipéridone, de préférence en milieu alcalin ou si $R_2$ représente un radical alcoyle, en milieu acide.

Dans des conditions préférentielles d'exécution, la réaction du produit de formule VI avec le chlorhydrate de 4-pipéridone est effectuée dans la potasse méthanolique 2N.

Des exemples d'une telle préparation figurent ci-après dans la partie expérimentale.

Les produits de formule VI dans laquelle R représente un radical méthylthio peuvent être préparés en faisant réagir un indole de formule:

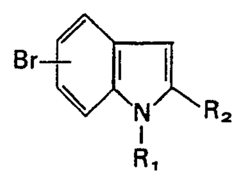

(VII)

avec le mercaptate cuivreux.

Un exemple d'une telle préparation est donné ci-après dans la partie expériementale.

Il va être donné maintenant, à titre non limitatif, des exemples de mise en œuvre de l'invention.

*Exemple 1:*

*Chlorhydrate de 5-méthylthio 3-(1-propyl 1,2,3,6-tétrahydro-4-pyridinyl) 1H-indole.*

On agite pendant 4 h, à température ambiante, une suspension de 7 g de 5-méthylthio 3-(1,2,3,6-tétrahydro-4-pyridinyl) 1H-indole, 6 g de carbonate de sodium et 4,2 $cm^3$ d'iodure de propyle dans 160 $cm^3$ de diméthylformamide. En fin de réaction on verse le mélange obtenu dans l'eau, filtre, lave à l'eau et sèche.

On isole ainsi 7,1 g d'un solide cristallisé jaune qui, par recristallisation dans l'isopropanol, donnent 6 g de cristaux jaunes fondant à 176° C.

*Préparation du chlorhydrate:*

On met en suspension 5 g de base dans 300 $cm^3$ d'acétate d'éthyle, refroidit à 0-5° C, rajoute une solution d'acétate d'éthyle chlorhydrique, filtre,

lave à l'acétate d'éthyle, sèche, recristallise dans un mélange de 500 $cm^3$ d'isopropanol et 100 $cm^3$ de méthanol et obtient 5,2 g de cristaux jaunes fondant à 236° C.

*Analyse* pour $C_{17}H_{22}N_2S$, HCl = 322,9

| | | | | | |
|---|---|---|---|---|---|
| Calculé: | C 63,24 | H 7,18 | N 8,67 | S 9,93 | Cl 10,98% |
| Trouvé: | C 63,4 | H 7,2 | N 8,5 | S 9,9 | Cl 11,1 % |

Le 5-méthylthio 3-(1,2,3,6-tétrahydro 4-pyridinyl) 1H-indole de départ peut être préparé comme suit:

a) 5-méthylthio 1H-indole.

On porte au reflux, sous agitation, pendant 5 h, 22,7 g de 5-bromo 1H-indole avec 230 $cm^3$ de quinoléïne, 34 $cm^3$ de pyridine anhydre et 16 g de méthylmercaptate cuivreux préparé selon Engelhardt (J. Med. Chem. // 329 (1968)); refroidit, précipite le mélange dans 1 l d'acide chlorhydrique 2N et 1 l d'acétate d'éthyle, filtre, décante, lave à l'acide chlorhydrique 2N et à l'eau salée, sèche, chasse les solvants sous pression réduite à 40° C et récupère 17,8 g de produit brut que l'on purifie par chromatographie sur silice en éluant par un mélange cyclohexanebenzène (1-1), on obtient 11,75 g de produit attendu.

*Spectre U.V. dans l'éthanol*

| | | | |
|---|---|---|---|
| Max.: | 225 nm | $E^{1\%}_{1\,cm}$ = 1812 | ε = 29 400 |
| Infl.: | 250 nm | = 710 | |
| Infl.: | 278 nm | = 249 | |
| Infl.: | 294 nm | = 188 | |
| Infl.: | 310 nm | = 98 | |

b) 5-méthylthio 3-(1,2,3,6-tétrahydro 4-pyridinyl) 1H-indole.

On agite sous atmosphère inerte le produit obtenu au stade précédent avec 22,1 g d'hydrate du chlorhydrate de 4-pipéridone et 108 $cm^3$ de potasse méthanolique 2N, porte la suspension obtenue pendant 16 h au reflux, refroidit, verse le mélange dans 1 l d'eau glacée, agite 15 min, filtre, lave à l'eau, sèche, recristallise dans un mélange d'acétate d'éthyle et de méthanol (10-3) et obtient 14,8 g de cristaux jaunes fondant à 210° C.

*Exemple 2:*

*Chlorhydrate de 5-nitro 3-(1-propyl 1,2,3,6-tétrahydropyridin-4-yl) 1H-indole.*

On agite pendant 5½ h, sous atmosphère inerte, à température ambiante, 11,2 g de chlorhydrate de 5-nitro 3-(1,2,3,6-tétrahydropyridin-4-yl) 1H-indole avec 140 $cm^3$ de diméthylformamide, 12,72 g de carbonate de sodium et 6,4 $cm^3$ d'iodure de propyle, dilue avec 300 $cm^3$ d'eau distillée, laisse cristalliser, agite une heure, essore, rince à l'eau puis avec 25 $cm^3$ d'éthanol à 50%, sèche et récupère 11,5 g de cristaux fondant à 164° C, que l'on chromatographie sur silice en éluant par un mélange chloroforme-acétone (8-2). On obtient 9,5 g de cristaux fondant à 172° C.

*Préparation du chlorhydrate:*

On met en suspension 6,13 g de la base obtenue dans 150 $cm^3$ d'éthanol, glace, ajoute de l'éthanol chlorhydrique jusqu'à pH = 1 sous agitation, agite 45 min sous atmosphère inerte, essore, rince à l'éthanol, sèche, récupère 6,90 g du produit cherché que l'on recristallise dans l'éthanol à 20% d'eau. On obtient 5,85 g de cristaux fondant à 240-242° C.

*Analyse* pour $C_{16}H_{19}N_3O_2$, HCl = 321,814

| | | | | |
|---|---|---|---|---|
| Calculé: | C 59,71 | H 6,26 | Cl 11,01 | N 13,05% |
| Trouvé: | C 59,6 | H 6,2 | Cl 11,1 | N 12,9 % |

Le chlorhydrate du 5-nitro 3-(1,2,3,6-tétrahydropyridin-4-yl) 1H-indole peut être préparé comme suit:

On porte au reflux sous atmosphère d'azote, pendant 3 h, 7,7 g de 5-nitro 1H-indole avec 225 $cm^3$ d'éthanol saturé d'acide chlorhydrique, 22,5 g de chlorhydrate de 4-pipéridone hydraté, puis agite 1 h à température ambiante et 1 h à 0° C, filtre, rince à l'éthanol glacé, à l'acétate d'éthyle, à l'éther et on obtient 14 g du produit brut cherché.

On recristallise les 14 g du produit obtenu dans 600 $cm^3$ de mélange méthanol-eau (1-1), laisse reposer une nuit à 0, +5° C, filtre, rince au méthanol, sèche et obtient 9,8 g du produit pur cherché fondant à 275° C.

*Analyse* pour $C_{13}H_{14}ClN_3O_2$ = 279,733

| | | | | |
|---|---|---|---|---|
| Calculé: | C 55,82 | H 5,04 | Cl 12,68 | N 15,02% |
| Trouvé: | C 56,0 | H 5,1 | Cl 12,8 | N 14,7 % |

*Exemple 3:*

*Chlorhydrate de 3-(1-propyl 1,2,3,6-tétrahydropyridin-4-yl) 1-H-indole-5-amine.*

En utilisant le même procédé que celui utilisé pour la préparation du produit des exemples 1 et 2, on a préparé le chlorhydrate de 3-(1-propyl 1,2,3,6-tétrahydropyridin-4-yl) 1-H-indole-5-amine à partir de 5-amino-indole.
F = 265° C.

*Analyse* pour $C_{12}H_{22}N_3Cl$ = 291,891

| | | | | |
|---|---|---|---|---|
| Calculé: | C 65,85 | H 7,59 | N 14,39 | Cl 12,15% |
| Trouvé: | C 65,9 | H 7,7 | N 14,1 | Cl 12,1 % |

*Exemple 4:*

*Chlorhydrate de 5-chloro 3-/1-(2-phénoxyéthyl)-1,2,3,6-tétrahydropyridin-4-yl/1H-indole.*

On agite pendant 2 h à 50° C sous atmosphère inerte, 8 g de 5-chloro 3-/1,2,3,6-tétrahydropyridin-4-yl/1H-indole en solution dans 80 $cm^3$ de diméthylformamide avec 7,5 g de carbonate de sodium et 9 g de β-bromophénétole, refroidit alors à température ambiante puis dilue lentement par 400 $cm^3$ d'eau distillée, agite encore 30 min, essore, lave à l'eau puis à l'aide d'un mélange éthanol-eau (1-1), sèche et récupère 14 g de produit brut.

On purifie 15,8 g de produit tel qu'obtenu ci-dessus par chromatographie sur colonne de silice en éluant à l'aide d'un mélange chloroforme-acétone-triéthylamine (6-3-1). On recristallise le produit obtenu dans l'éthanol et obtient 11,53 g d'un produit fondant à 172° C.

*Préparation du chlorhydrate:*

On met en suspension le produit obtenu ci-dessus dans 500 cm³ d'éthanol, ajoute, goutte à goutte, une solution d'éthanol chlorhydrique jusqu'à pH acide, redissout à chaud, filtre, concentre de moitié, cristallise, essore, lave à l'éthanol et sèche. On récupère 12,1 g de produit fondant à 190° C que l'on recristallise dans l'éthanol pour obtenir 9,25 g de produit fondant à 180° C puis à 220° C.

*Analyse* pour $C_{21}H_{22}Cl_2N_2O$ = 389,3

| | | | |
|---|---|---|---|
| Calculé: | C 64,78 | H 5,70 | N 7,20% |
| Trouvé: | C 64,1 | H 6,0 | N 6,9 % |

Le 5-chloro 3-(1,2,3,6-tétrahydropyridin-4-yl) 1H-indole de départ peut être préparé comme décrit dans le brevet français nº 2362628.

*Exemple 5:*

*Oxalate neutre de 4-(1H-indol-3-yl)-3,6-dihydro-1-(2H)pyridin éthanol.*

*Stade A: 3-/1-/2-(2-tétrahydropyranyloxy)-éthyl/1,2,3,6-tétrahydropyridin-4-yl/1H-indole.*

On porte pendant 5½ h au reflux sous agitation et atmosphère inerte, 6,93 g de 3-(1,2,3,6-tétrahydropyridin-4-yl) 1H-indole (décrit dans le brevet français nº 2362628), dissous à 100° C dans 70 cm³ d'isobutylméthylcétone avec 11,13 g de carbonate de sodium et 14 cm³ de 2-/(2-chloroéthyl) oxy/tétrahydro 2H-pyran, laisse ensuite refroidir et verse dans l'eau glacée.

On extrait à l'acétate d'éthyle, lave à l'eau et à l'eau salée, sèche, évapore à sec, récupère 9,9 g de produit cristallisé que l'on purifie par chromatographie sur silice (éluant: chloroformeacétonetriéthylamine 85-10-5) et obtient 7,85 g de cristaux fondant à 135° C.

*Stade B: Oxalate neutre de 4-(1H-indol-3-yl) 3,6-dihydro 1-(2H) pyridinéthanol.*

On introduit le produit obtenu au stade précédent dans 156 cm³ d'éthanol à 95°, ajoute 15,56 cm³ d'acide chlorhydrique 6N, agite pendant 3½ h sous atmosphère inerte, verse le mélange obtenu dans un litre d'eau, ajoute 10 cm³ de lessive de soude, filtre et lave à l'eau les cristaux ainsi obtenus. On sèche sous pression réduite et obtient 4,068 g de cristaux fondant à 164-165° C.

*Préparation de l'oxalate neutre:*

On dissout 3,815 g du produit obtenu ci-dessus dans 200 cm³ d'éthanol, ajoute 992 mg d'acide oxalique, redissout les cristaux obtenus dans 1½ l d'éthanol au reflux, filtre à chaud, concentre la solution, laisse cristalliser lentement, essore, rince à l'éthanol et obtient 3,245 g du produit cherché fondant à 197-200° C.

*Analyse* pour $C_{32}H_{38}N_4O_6$ = 574,683

| | | | |
|---|---|---|---|
| Calculé: | C 66,88 | H 6,66 | N 9,74% |
| Trouvé: | C 66,6 | H 6,6 | N 9,6 % |

*Exemple 6:*

*Chlorhydrate de 3-/1-cyclopropylméthyl) 1,2,3,6-tétrahydropyridin-4-yl 5-nitro 1H-indole.*

On agite pendant 23 h sous atmosphère inerte à environ 70° C, 11,88 g de chlorhydrate de 5-nitro 3-(1,2,3,6-tétrahydropyridin-4-yl) 1H-indole avec 148 cm³ de diméthylformamide, 12,72 g de carbonate de sodium et 4,8 cm³ de chlorométhylcyclopropane, laisse refroidir, ajoute 450 cm³ d'eau distillée, agite une heure, essore, rince à l'eau, sèche et récupère 10,8 g de produit brut que l'on chromatographie sur silice (éluant: chloroformeacétonetriéthylamine 6-3-1). On obtient 8,3 g du produit attendu fondant à 187-188° C.

*Préparation du chlorhydrate*

On met en suspension 8,7 g de la base telle qu'obtenue au stade précédent dans 130 cm³ d'éthanol, glace, ajoute de l'éthanol chlorhydrique jusqu'à pH1 sous agitation, agite 2 h sous atmosphère inerte, essore, rince à l'éthanol, sèche, récupère 9,55 g du produit recherché que l'on recristallise dans le méthanol. On obtient 7,2 g de cristaux fondant à 253-255° C.

*Analyse* pour $C_{17}H_{20}ClN_3O_2$ = 333,826

| | | | | |
|---|---|---|---|---|
| Calculé: | C 61,16 | H 6,03 | Cl 10,62 | N 12,58% |
| Trouvé: | C 61,5 | H 6,1 | Cl 10,9 | N 12,4 % |

*Exemple 7:*

*Oxalate neutre de 4-(5-chloro 1H-indol-3-yl) 1,2,3,6-tétrahydro 1-(2H) pyridinpropanol.*

*Stade A: 3-/1-/3-(2-tétrahydropyranyloxy) propyl/1,2,3,6-tétrahydropyridin-4-yl/1H-indole.*

On chauffe à 100-105° C pendant 24 h sous agitation et atmosphère inerte 11,6 g de 5-chloro 3-(1,2,3,6-tétrahydropyridin-4-yl) 1H-indole dissous dans 120 cm³ d'isobutylméthylcétone avec 15,9 g de carbonate de sodium et 22 cm³ de 3-/(3-chloropropyl)oxy/tétrahydro 2H-pyran, laisse ensuite refroidir et verse dans 500 cm³ d'eau.

On agite pendant une heure, extrait à l'acétate d'éthyle, lave à l'eau et à l'eau salée, sèche, évapore à sec, récupère 29,6 g d'un produit cristallisé que l'on purifie par chromatographie sur silice (éluant: chloroformeacétonetriéthylamine 6-3-1 et obtient 13,1 g de cristaux fondant à 161° C.

*Stade B: Oxalate neutre de 4-(5-chloro 1H-indol-3-yl) 1,2,3,6-tétrahydro 1-(2H) pyridinpropanol.*

On introduit le produit obtenu au stade précédent dans 290 cm³ d'éthanol à 95°, ajoute 71,5 cm³ d'acide chlorhydrique 2N, agite pendant

4 h sous atmosphère inerte, verse le mélange obtenu dans 500 $cm^3$ d'eau, alcalinise avec de la lessive de soude, agite une heure, filtre et lave à l'eau les cristaux ainsi obtenus. On sèche sous pression réduite et obtient 7,7 g de cristaux fondant à 161-162° C.

*Préparation de l'oxalate neutre*

On dissout 7,7 g du produit obtenu ci-dessus dans 77 $cm^3$ d'éthanol, ajoute 1,66 g d'acide oxalique en solution dans 16 $cm^3$ d'éthanol, agite sous atmosphère inerte, essore, rince à l'éthanol, recristallise dans l'eau distillée, essore, rince, sèche et obtient 7,11 g du produit recherché fondant à 195° C et 217° C.

*Analyse* pour $C_{34}H_{40}Cl_2N_4O_6$ = 671,627

| | | | | |
|---|---|---|---|---|
| Calculé: | C 60,80 | H 6,00 | Cl 10,55 | N 8,34% |
| Trouvé: | C 60,7 | H 6,0 | Cl 10,8 | N 8,2 % |

*Exemple 8:*

On a préparé des comprimés répondant à la formule:

– Chlorhydrate de 5-nitro 3-(1-propyl 1,2,3,6-tétrahydropyridin-4-yl) 1H-indole 30 mg
– Excipient q.s. pour un comprimé terminé à 100 mg

(Détail de l'excipient: lactose, amidon, talc, stéarate de magnésium).

*Exemple 9:*

On a préparé des comprimés répondant à la formule:

– Oxalate neutre de 4-(1H-indol-3-yl) 3,6-dihydro-1-(2H) pyridinéthanol 30 mg
– Excipient q.s. pour un comprimé terminé à 100 mg

(Détail de l'excipient: lactose, amidon, talc, stéarate de magnésium).

*Exemple 10:*

On a préparé des comprimés répondant à la formule:

– Chlorhydrate de 5-méthylthio 3-(1-propyl 1,2,3,6-tétrahydro 4-pyridinyl) 1H-indole 50 mg
– Excipient q.s. pour un comprimé terminé à 100 mg

(Détail de l'excipient: lactose, amidon, talc, stéarate de magnésium).

*Etude pharmacologique*

1. *Antagonisme des stéréotypies à l'amphétamine:*

Les essais sont réalisés sur des lots de 5 rats mâles de 150 à 180 g. Chaque animal est placé individuellement dans une boîte (29×25×17 cm) contenant quelques débris de frisure de bois.

Un délai d'une demi-heure est observé entre l'administration du composé étudié et l'injection, par voie intrapéritonéale, de 8 mg/kg de sulfate de dexamphétamine. Le comportement des animaux est noté ensuite toutes les ½ h pendant 5 h avec la cotation préconisée par Halliwell et Coll. («Brit. J. Pharmacol.» 1964, *23*, 330-350). L'animal est endormi (0), il est éveillé mais immobile (1), il tourne dans la cage (2), il en renifle le couvercle (3), il en lèche les parois (4), il touche les copeaux ou les barreaux de la cage avec les dents (5), il mord les copeaux ou les barreaux de la cage (6).

L'intensité des stéréotypies est exprimée sous la forme d'un score compris entre 0 et 30 correspondant à la somme des valeurs obtenues sur les 5 rats d'un lot à chaque temps. La somme des scores relevés en 5 h est calculée.

Les composés sont administrés par voie intrapéritonéale.

La dose des composés qui diminue d'environ 50% la somme des scores en 5 h est de 20 mg/kg pour le composé de l'exemple 4 et de 25 mg/kg pour le composé de l'exemple 2.

2. *Antagonisme à l'égard des stéréotypies à l'apomorphine.*

Les essais sont réalisés sur des lots de 5 rats selon un protocole inspiré de Janssen et Coll. («Arzneim. Forsch.» 1965, *15*, 104-117; 1967, *17*, 841-854). Chaque animal est placé individuellement dans une boîte en plexiglas (20×10×10 cm; Nicolet) dont le fond est recouvert d'une mince couche de frisure de bois.

Une dose de 1,5 mg/kg de chlorhydrate d'apomorphine est injectée par voie intraveineuse, ½ h après l'administration intrapéritonéale du composé étudié.

Les animaux sont observés pendant 1 min, 15 min après l'injection de l'apomorphine. Les mouvements stéréotypes de la sphère buccale sont évalués selon Boissier et Simon («Thérapie», 1970, *25*, 933-949): pas de réaction caractéristique (0), quelques reniflements, léchages et mâchonnements (1), reniflements intenses et léchages continus (2), mâchonnements continus (3).

L'intensité des stéréotypies est exprimée sous la forme d'un score compris entre 0 et 15, correspondant à la somme des valeurs obtenues sur les 5 rats d'un lot, 15 min après l'injection de l'apomorphine.

La dose des composés qui réduit d'environ 50% la somme des scores est de 2 mg/kg pour le composé de l'exemple 2 et de 7 mg/kg pour le composé de l'exemple 1.

3. *Antagonisme de la catalepsie induite par la prochlorpemazine.*

Les essais sont réalisés sur des lots de 5 rats mâles de 100 g environ.

Le composé étudié est administré par voie intrapéritonéale simultanément avec une dose de 15 mg/kg de prochlorpemazine par voie intrapéritonéale.

La catalepsie est appréciée toutes les heures, pendant 7 h, suivant le test de croisement des pattes homolatérales (Boissier, Simon, «Thérapie», 1963, *18*, 1257-1277) avec la cotation suivante: l'animal refuse le croisement des pattes antérieures avec les pattes postérieures homolaté-

rales (0), il accepte le croisement recherché seulement d'un côté (0, 5), il accepte le croisement des deux côtés (1).

Le composé de l'exemple 1 s'oppose à la catalepsie induite par la prochlorpemazine à partir de la dose de 10 mg/kg; celui de l'exemple 5, à partir de la dose de 20 mg/kg.

Cette action anticataleptique est particulièrement intéressante car elle se manifeste à des doses inférieures à celles auxquelles les produits exercent, par eux-mêmes, un effet cataleptique.

4. *Activité antiémétique.*

L'antagonisme vis-à-vis des vomissements provoqués par l'apomorphine est étudié chez le chien (Chen et Ensor «J. Pharmac. exp. Therap.» 1959, *93*, 245-250).

Le nombre de vomissements provoqué par une injection sous-cutanée de 0,1 mg/kg de chlorhydrate d'apomorphine est déterminé sur chaque animal 8 jours avant l'essai.

Le composé étudié, mis en solution aqueuse, est administré par voie sous-cutanée à des doses variables, une ½ h avant le chlorhydrate d'apomorphine.

Le composé de l'exemple 2 réduit d'environ 50% les vomissements provoqués par l'apomorphine à la dose de 0,01 mg/kg, le composé de l'exemple 1, à la dose de 0,08 mg/kg.

5. *Etude de la toxicité aiguë.*

La toxicité aiguë est déterminée sur des lots de 10 souris, pesant 20 g environ, auxquelles on administre, par voie intrapéritonéale, des doses croissantes du composé étudié.

La mortalité est relevée 48 h après l'administration du composé.

La dose létale 50 ($DL_{50}$) du composé de l'exemple 4 est supérieure à 400 mg/kg; celle du composé de l'exemple 5 est égale à 300 mg/kg; celle des exemples 1 et 2 est égale à 200 mg/kg.

**Revendications pour les Etats contractants:**
BE, GE, LI, DE, FR, GB, IT, W, NL, SE

1. Nouveaux dérivés N-substitués du tétrahydropyridinylindole et de leurs sels d'addition avec les acides minéraux ou organiques, caractérisés en ce qu'ils répondent à la formule générale:

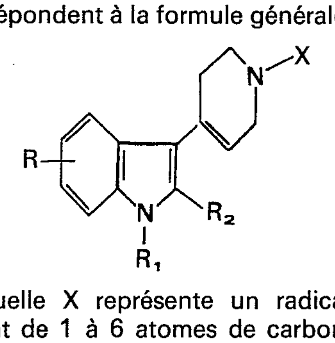

(I)

dans laquelle X représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone, hydroxyalcoyle dans lequel le radical alcoyle renferme de 2 à 5 atomes de carbone ou phénoxyalcoyle dans lequel le radical alcoyle renferme de 1 à 5 atomes de carbone, R représente un atome d'hydrogène ou d'halogène ou un radical alcoxy renfermant de 1 à 3 atomes de carbone, nitro, amino, trifluorométhyl ou méthyltio, $R_1$ et $R_2$ représentent un atome d'hydrogène ou un radical alcoyle renfermant de 1 à 3 atomes de carbone, étant entendu que si R représente un atome d'hydrogène, d'halogène ou un radical alcoxy renfermant de 1 à 3 atomes de carbone, X représente obligatoirement un radical hydroxyalcoyle ou phénoxyalcoyle tels que définis ci-dessus.

2. Dérivés N-substitués du tétrahydropyridinylindole et de leurs sels d'addition avec les acides minéraux ou organiques, répondant à la formule I selon la revendication 1, caractérisés en ce que X représente un radical hydroxyalcoyle dans lequel le radical alcoyle renferme de 2 à 5 atomes de carbone et R, $R_1$ et $R_2$ ont la signification déjà indiquée.

3. Dérivés N-substitués du tétrahydropyridinylindole et de leurs sels d'addition avec les acides minéraux ou organiques, répondant à la formule I selon la revendication 1, caractérisés en ce que X représente un radical phénoxyalcoyle dans lequel le radical alcoyle renferme de 1 à 5 atomes de carbone et R, $R_1$ et $R_2$ ont la signification déjà indiquée.

4. Dérivés N-substitués du tétrahydropyridinylindole et de leurs sels d'addition avec les acides minéraux ou organiques, répondant à la formule I selon la revendication 1, caractérisés en ce que X représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone et R, $R_1$ et $R_2$ ont la signification déjà indiquée.

5. Le chlorhydrate de 5-nitro 3-(1-propyl 1,2,3,6-tétrahydropyridin-4-yl) 1H-indole.

6. Procédé de préparation des dérivés de formule I selon la revendication 1 ainsi que de leurs sels d'addition avec les acides minéraux ou organiques, dans laquelle X représente un radical hydroxyalcoyle dans lequel le radical alcoyle renferme de 2 à 5 atomes de carbone et R, $R_1$ et $R_2$ ont la signification déjà indiquée, caractérisé en ce que l'on fait réagir un produit de formule:

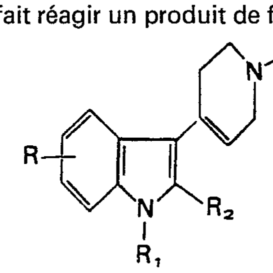

(II)

dans laquelle R, $R_1$ et $R_2$ ont la signification déjà énoncée, avec un halogénure de formule:

Hal−X′ (III)

dans laquelle Hal représente un atome de chlore, de brome ou d'iode et X′ représente un radical tétrahydropyranyloxyalcoyle de formule:

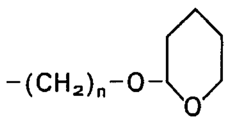

(IV)

dans laquelle n représente un nombre entier égal à 2,3,4 ou 5, pour obtenir un dérivé de formule:

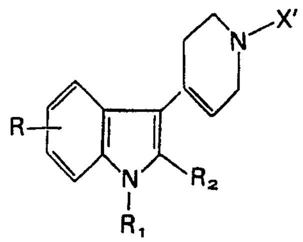

(V)

dans laquelle X′, R, $R_1$ et $R_2$ ont la signification déjà indiquée, produit de formule V que l'on hydrolyse pour obtenir un produit de formule I dans laquelle X représente un radical hydroxyalcoyle dans lequel le radical alcoyle renferme de 2 à 5 atomes de carbone et R, $R_1$ et $R_2$ ont la signification déjà indiquée, que l'on isole et, le cas échéant, traite par un acide pour en former le sel.

7. Procédé de préparation des dérivés de formule I selon la revendication 1 ainsi que de leurs sels d'addition avec les acides minéraux ou organiques dans laquelle X représente un radical phénoxyalcoyle dans lequel le radical alcoyle renferme de 1 à 5 atomes de carbone, un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone et R, $R_1$ et $R_2$ ont la signification déjà énoncée, caractérisé en ce que l'on fait réagir un produit de formule:

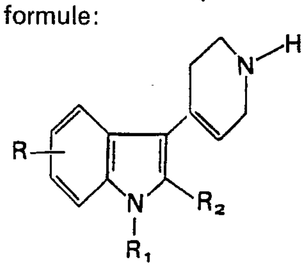

(II)

dans laquelle R, $R_1$ et $R_2$ ont la signification déjà indiquée, avec un halogénure de formule générale:

Hal−X″ (III′)

dans laquelle Hal représente un atome de chlore, de brome ou d'iode et X″ représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone ou phénoxyalcoyle dans lequel le radical alcoyle renferme de 1 à 5 atomes de carbone, pour obtenir un produit de formule I dans laquelle R, $R_1$, $R_2$ et X ont la signification déjà indiquée, que l'on isole et, le cas échéant, traite par un acide pour en former le sel.

8. Médicaments, caractérisés en ce qu'ils sont constitués par les nouveaux dérivés N-substitués du tétrahydropyridinylindole, tels que définis par la formule I de la revendication 1, ainsi que par leurs sels d'addition avec les acides pharmaceutiquement acceptables.

9. Médicaments, caractérisés en ce qu'ils sont constitués par les nouveaux dérivés N-substitués du tétrahydropyridinylindole, tels que définis dans l'une des revendications 2, 3 ou 4, ainsi que par leurs sels d'addition avec les acides pharmaceutiquement acceptables.

10. Médicament, caractérisé en ce qu'il est constitué par le dérivé N-substitué du tétrahydropyridinylindole, tel que défini à la revendication 5.

11. Compositions pharmaceutiques, caractérisées en ce qu'elles renferment, à titre de principe actif, l'un au moins des médicaments, tels que définis à l'une des revendications 8, 9 ou 10.

**Revendications pour l'Etat contractant:** AT

1. Procédé de préparation des nouveaux dérivés N-substitués du tétrahydropyridinylindole et de leurs sels d'addition avec les acides minéraux ou organiques, répondant à la formule générale:

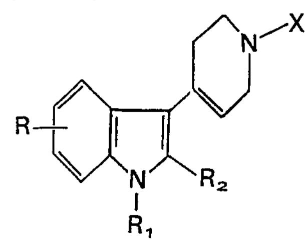

(I)

dans laquelle X représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone, hydroxyalcoyle dans lequel le radical alcoyle renferme de 2 à 5 atomes de carbone ou phénoxyalcoyle dans lequel le radical alcoyle renferme de 1 à 5 atomes de carbone, R représente un atome d'hydrogène ou d'halogène ou un radical alcoxy renfermant de 1 à 3 atomes de carbone, nitro, amino, trifluorométhyl ou méthylthio, $R_1$ et $R_2$ représentent un atome d'hydrogène ou un radical alcoyle renfermant de 1 à 3 atomes de carbone, étant entendu que si R représente un atome d'hydrogène, d'halogène ou un radical alcoxy renfermant de 1 à 3 atomes de carbone, X représente obligatoirement un radical hydroxyalcoyle ou phénoxyalcoyle tels que définis ci-dessus, caractérisé en ce que:

a) pour préparer les dérivés de formule I, ainsi

que leurs sels d'addition avec les acides minéraux ou organiques, dans laquelle X représente un radical hydroxyalcoyle dans lequel le radical alcoyle renferme de 2 à 5 atomes de carbone et R, $R_1$ et $R_2$ ont la signification déjà indiquée, l'on fait réagir un produit de formule:

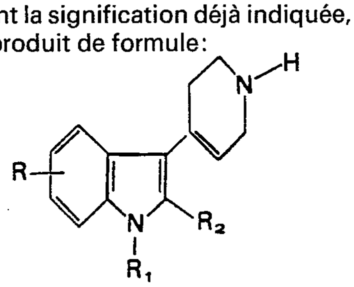

(II)

dans laquelle R, $R_1$ et $R_2$ ont la signification déjà énoncée, avec un halogénure de formule:

Hal−X' (III)

dans laquelle Hal représente un atome de chlore, de brome ou d'iode et X' représente un radical tétrahydropyranyloxy alcoyle de formule:

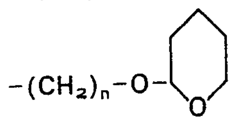

(IV)

dans laquelle n représente un nombre entier égal à 2,3,4 ou 5, pour obtenir un dérivé de formule:

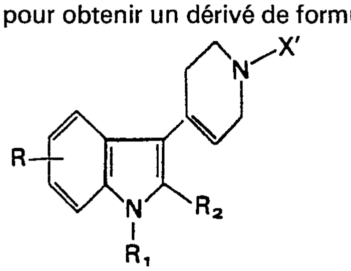

(V)

dans laquelle X', R, $R_1$ et $R_2$ ont la signification déjà indiquée, produit de formule V que l'on hydrolyse pour obtenir un produit de formule I dans laquelle X représente un radical hydroxyalcoyle dans lequel le radical alcoyle renferme de 2 à 5 atomes de carbone et R, $R_1$ et $R_2$ ont la signification déjà indiquée, que l'on isole et, le cas échéant, traite par un acide pour en former le sel;

b) pour préparer les dérivés de formule I, ainsi que leurs sels d'addition avec les acides minéraux ou organiques dans laquelle X représente un radical phénoxyalcoyle dans lequel le radical alcoyle renferme de 1 à 5 atomes de carbone, un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone et R, $R_1$ et $R_2$ ont la signification déjà énoncée, l'on fait réagir un produit de formule:

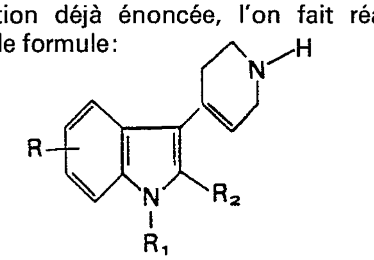

(II)

dans laquelle R, $R_1$ et $R_2$ ont la signification déjà indiquée, avec un halogénure de formule générale:

Hal−X'' (III')

dans laquelle Hal représente un atome de chlore, de brome ou d'iode et X'' représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone ou phénoxyalcoyle dans lequel le radical alcoyle renferme de 1 à 5 atomes de carbone, pour obtenir un produit de formule I dans laquelle R, $R_1$, $R_2$ et X ont la signification déjà indiquée, que l'on isole et, le cas échéant, traite par un acide pour en former le sel.

2. Procédé selon la revendication 1, pour la préparation des dérivés de formule I, ainsi que de leurs sels d'addition avec les acides minéraux ou organiques, dans laquelle X représente un radical hydroxyalcoyle dans lequel le radical alcoyle renferme de 2 à 5 atomes de carbone et R, $R_1$ et $R_2$ ont la signification déjà indiquée, caractérisé en ce que l'on fait réagir un produit de formule:

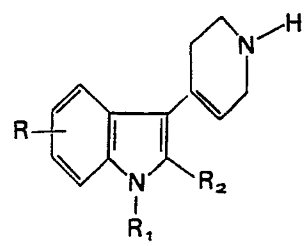

(II)

dans laquelle R, $R_1$ et $R_2$ ont la signification déjà énoncée, avec un halogénure de formule:

Hal−X' (III)

dans laquelle Hal représente un atome de chlore, de brome ou d'iode et X' représente un radical tétrahydropyranyloxyalcoyle de formule:

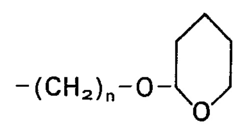

(IV)

dans laquelle n représente un nombre entier égal à 2, 3, 4 ou 5, pour obtenir un dérivé de formule:

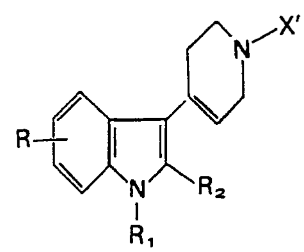

(V)

dans laquelle X', R, $R_1$ et $R_2$ ont la signification déjà indiquée, produit de formule V que l'on hydrolyse pour obtenir un produit de formule I dans laquelle X représente un radical hydroxyalcoyle, dans lequel le radical alcoyle renferme de 2 à 5 atomes de carbone et R, $R_1$ et $R_2$ ont la

signification déjà indiquée, que l'on isole et, le cas échéant, traite par un acide pour en former le sel.

3. Procédé selon la revendication 1, pour la préparation des dérivés de formule I ainsi que de leurs sels d'addition avec les acides minéraux ou organiques, dans laquelle X représente un radical phénoxyalcoyle dans lequel le radical alcoyle renferme de 1 à 5 atomes de carbone, un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone ou aralcoyle renfermant de 7 à 12 atomes de carbone et R, $R_1$ et $R_2$ ont la signification déjà énoncée, caractérisé en ce que l'on fait réagir un produit de formule:

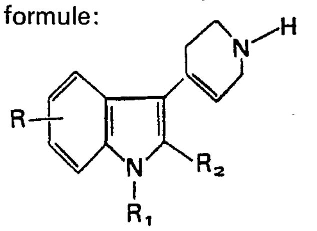

(II)

dans laquelle R, $R_1$ et $R_2$ ont la signification déjà indiquée, avec un halogénure de formule générale:

Hal–X″ (III′)

dans laquelle Hal représente un atome de chlore, de brome ou d'iode et X″ représente un radical alcoyle renfermant de 1 à 6 atomes de carbone, cycloalcoyle renfermant 5 ou 6 atomes de carbone, cycloalcoylalcoyle renfermant de 4 à 7 atomes de carbone, alcényle renfermant de 2 à 5 atomes de carbone, alcynyle renfermant de 3 à 5 atomes de carbone, aralcoyle renfermant de 7 à 12 atomes de carbone ou phénoxyalcoyle dans lequel le radical alcoyle renferme de 1 à 5 atomes de carbone, pour obtenir un produit de formule I dans laquelle R, $R_1$, $R_2$ et X ont la signification déjà indiquée, que l'on isole et, le cas échéant, traite par un acide pour en former le sel.

4. Procédé selon la revendication 1 ou 3, caractérisé en ce que l'on utilise au départ un dérivé de formule II dans laquelle R est un radical 5-nitro et $R_1$ et $R_2$ représentent un atome d'hydrogène et un halogénure de formule III′ dans laquelle X″ représente un radical propyle.

**Patentansprüche für die Vertragsstaten:** BE, CH, DE, FR, GB, IT, LI, LU, NL, SE

1. Neue N-substituierte Tetrahydropyridinylindolderivate und deren Additionssalzen mit Mineral- oder organischen Säuren, dadurch gekennzeichnet, das sie der allgemeinen Formel:

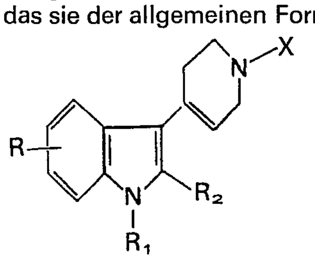

(I)

entsprechen, in der X einen Alkylrest mit 1 bis 6 Kohlenstoffatomen, einen Cycloalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen, einen Aralkylrest mit 7 bis 12 Kohlenstoffatomen, einen Hydroxyalkylrest, worin der Alkylrest 2 bis 5 Kohlenstoffatomen umfasst, oder einen Phenoxyalkylrest, worin der Alkylrest 1 bis 5 Kohlenstoffatome umfasst, bedeutet,

R ein Wasserstoff- oder Halogenatom oder einen Alkoxyrest mit 1 bis 3 Kohlenstoffatomen, die Nitro-, Amino-, Trifluormethyl- oder Methylthiogruppe bedeutet, und

$R_1$ und $R_2$ ein Wasserstoffatom oder einen Alkylrest mit 1 bis 3 Kohlenstoffatomen bedeuten, wobei, wenn R ein Wasserstoff- oder Halogenatom oder einen Alkoxyrest mit 1 bis 3 Kohlenstoffatomen bedeutet, X obligatorisch einen Hydroxyalkyl- oder Phenoxyalkylrest wie vorstehend definiert darstellt.

2. N-substituierte Tetrahydropyridinylindolderivate und deren Additionssalze mit Mineral- oder organischen Säuren der Formel (I) gemäss dem Anspruch 1, dadurch gekennzeichnet, dass X einen Hydroxyalkylrest bedeutet, worin der Alkylrest 2 bis 5 Kohlenstoffatome umfasst, und R, $R_1$ und $R_2$ die vorstehend angegebene Bedeutung besitzen.

3. N-substituierte Tetrahydropyridinylindolderivate und deren Additionssalze mit Mineral- oder organischen Säuren der Formel (I) gemäss dem Anspruch 1, dadurch gekennzeichnet, dass X einen Phenoxyalkylrest bedeutet, worin der Alkylrest 1 bis 5 Kohlenstoffatome umfasst, und R, $R_1$ und $R_2$ die angegebene Bedeutung besitzen.

4. N-substituierte Tetrahydropyridinylindolderivate und deren Additionssalze mit Mineral- oder organischen Säuren der Formel (I) gemäss dem Anspruch 1, dadurch gekennzeichnet, dass X einen Alkylrest mit 1 bis 6 Kohlenstoffatomen, einen Cyclohalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen oder einen Aralkylrest mit 7 bis 12 Kohlenstoffatomen bedeutet, und R, $R_1$ und $R_2$ die angegebene Bedeutung besitzen.

5. 5-Nitro-3-(1-propyl-1,2,3,6-tetrahydropyridin-4-yl)-[1H]-indolhydrochlorid.

6. Verfahren zur Herstellung der Derivate der Formel (I) gemäss dem Anspruch 1 sowie von deren Additionssalzen mit Mineral- oder organischen Säuren, worin X einen Hydroxyalkylrest, in dem der Alkylrest 2 bis 5 Kohlenstoffatome umfasst, bedeutet, und R, $R_1$ und $R_2$ die angegebene Bedeutung besitzen, dadurch gekennzeichnet, dass man ein Produkt der Formel:

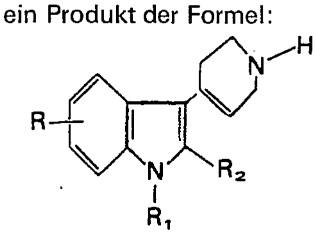

(II)

worin R, $R_1$ und $R_2$ die angegebene Bedeutung besitzen, mit einem Halogenid der Formel:

Hal–X' (III)

worin Hal ein Chlor-, Brom- oder Jodatom bedeutet und X' einen Tetrahydropyranyloxyalkylrest der Formel:

$-(CH_2)_n-O-$ 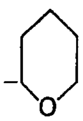 (IV)

bedeutet, worin n eine ganze Zahl entsprechend 2, 3, 4 oder 5 darstellt, umsetzt, um ein Derivat der Formel:

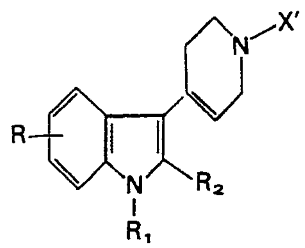

(V)

zu erhalten, worin X', R, $R_1$ und $R_2$ die angegebene Bedeutung besitzen, das Produkt der Formel(V) hydrolysiert, um ein Produkt der Formel I zu erhalten, worin X einen Hydroxyalkylrest darstellt, in dem der Alkylrest 2 bis 5 Kohlenstoffatome umfasst, und R, $R_1$ und $R_2$ die angegebene Bedeutung besitzen, welches man isoliert und gewünschtenfalls mit einer Säure behandelt, um hieraus das Salz zu bilden.

7. Verfahren zur Herstellung der Derivate der Formel (I) gemäss dem Anspruch 1 sowie von deren Additionssalzen mit Mineral- oder organischen Säuren, worin X einen Phenoxyalkylrest, in dem der Alkylrest 1 bis 5 Kohlenstoffatome umfasst, einen Alkylrest mit 1 bis 6 Kohlenstoffatomen, einen Cycloalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen oder einen Aralkylrest mit 7 bis 12 Kohlenstoffatomen bedeutet, und R, $R_1$ und $R_2$ die angegebene Bedeutung besitzen, dadurch gekennzeichnet, dass man ein Produkt der Formel:

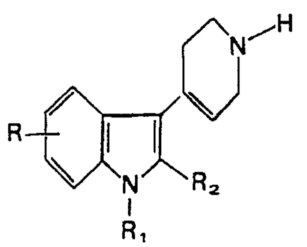

(II)

worin R, $R_1$ und $R_2$ die angegebene Bedeutung besitzen, mit einem Halogenid der allgemeinen Formel:

Hal–X'' (III')

worin Hal ein Chlor-, Brom- oder Jodatom bedeutet, und X'' einen Alkylrest mit 1 bis 6 Kohlenstoffatomen, einen Cycloalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen, einen Aralkylrest mit 7 bis 12 Kohlenstoffatomen oder einen Phenoxyalkylrest, in dem der Alkylrest 1 bis 5 Kohlenstoffatome umfasst, bedeutet, umsetzt, um ein Produkt der Formel (I) zu erhalten, worin R, $R_1$, $R_2$ und X die angegebene Bedeutung besitzen, welches man isoliert und gewünschtenfalls mit einer Säure behandelt, um hieraus das Salz zu bilden.

8. Arzneimittel, dadurch gekennzeichnet, dass sie aus den neuen N-substituierten Tetrahydropyridinylindolderivaten der Formel (I) gemäss dem Anspruch 1 sowie aus deren Additionssalzen mit pharmazeutisch verträglichen Säuren bestehen.

9. Arzneimittel, dadurch gekennzeichnet, dass sie aus den neuen N-substituierten Tetrahydropyridinylindolderivaten gemäss einem der Ansprüche 2, 3 oder 4 sowie aus deren Additionssalzen mit pharmazeutisch verträglichen Säuren bestehen.

10. Arzneimittel, dadurch gekennzeichnet, dass es aus dem N-substituierten Tetrahydropyridinylindolderivat gemäss dem Anspruch 5 besteht.

11. Pharmazeutische Zusammensetzungen, dadurch gekennzeichnet, dass sie als Wirkstoff zumindest eines der Arzneimittel gemäss der Ansprüche 8, 9 oder 10 umfassen.

**Patentansprüche für den Vertragsstaat:** AT

1. Verfahren zur Herstellung neuer N-substituierter Tetrahydropyridinylindolderivate und von deren Additionssalzen mit Mineral- oder organischen Säuren der allgemeinen Formel:

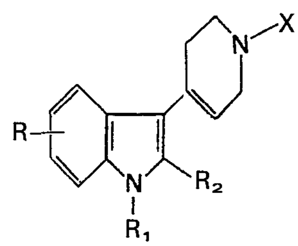

(I)

in der X einen Alkylrest mit 1 bis 6 Kohlenstoffatomen, einen Cycloalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen, einen Aralkylrest mit 7 bis 12 Kohlenstoffatomen, einen Hydroxyalkylrest, worin der Alkylrest 2 bis 5 Kohlenstoffatomen umfasst, oder einen Phenoxyalkylrest, worin der Alkylrest 1 bis 5 Kohlenstoffatome umfasst, bedeutet,

R ein Wasserstoff- oder Halogenatom oder einen Alkoxyrest mit 1 bis 3 Kohlenstoffatomen, die Nitro-, Amino-, Trifluormethyl- oder Methylthiogruppe bedeutet, und

$R_1$ und $R_2$ ein Wasserstoffatom oder einen Alkylrest mit 1 bis 3 Kohlenstoffatomen bedeuten, wobei, wenn R ein Wasserstoffatom, ein Halogenatom oder einen Alkoxyrest mit 1 bis 3 Kohlenstoffatomen bedeutet, X obligatorisch einen Hydroxyalkyl- oder Phenoxyalkylrest wie vorstehend definiert darstellt, dadurch gekennzeichnet, dass

a) man zur Herstellung der Derivate der Formel I sowie von deren Additionssalzen mit Mineral- oder organischen Säuren, worin X einen Hydroxyalkylrest, in dem der Alkylteil 2 bis 5 Kohlenstoffatome umfasst, darstellt, und R, $R_1$ und $R_2$ die angegebene Bedeutung besitzen, ein Produkt der Formel:

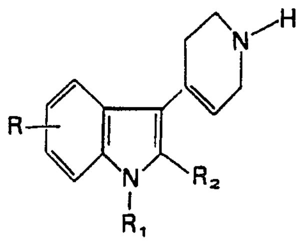
(II)

worin R, $R_1$ und $R_2$ die angegebenen Bedeutung besitzen, mit einem Halogenid der Formel:

Hal−X' (III)

worin Hal ein Chlor-, Brom- oder Jodatom bedeutet und X' einen Tetrahydropyranyloxyalkylrest der Formel:

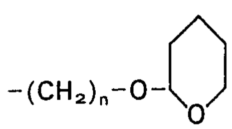
(IV)

darstellt, worin n eine ganze Zahl entsprechend 2, 3, 4 oder 5 bedeutet, umsetzt, um ein Derivat der Formel:

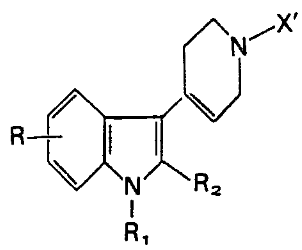
(V)

zu erhalten, worin X', R, $R_1$ und $R_2$ die angegebene Bedeutung besitzten, das Produkt der Formel V hydrolysiert, um ein Produkt der Formel I zu erhalten, worin X einen Hydroxyalkylrest, in dem der Alkylrest 2 bis 5 Kohlenstoffatome umfasst, bedeutet, und R, $R_1$ und $R_2$ die angegebene Bedeutung besitzen, welches man isoliert und gewünschtenfalls mit einer Säure behandelt, um hieraus das Salz zu bilden,

b) man zur Herstellung der Derivate der Formel (I) sowie von deren Additionssalzen mit Mineral- oder organischen Säuren, worin X einen Phenoxyalkylrest, in dem der Alkylteil 1 bis 5 Kohlenstoffatome umfasst, einen Alkylrest mit 1 bis 6 Kohlenstoffatomen, einen Cycloalkylrest mit 5 oder 6 Kohlenstoffatomen, einen Cycloalkylrest mit 4 bis 7 Kohlenstoffatomen, einen Alkenylrest mit 2 bis 5 Kohlenstoffatomen, einen Alkinylrest mit 3 bis 5 Kohlenstoffatomen oder einen Aralkylrest mit 7 bis 12 Kohlenstoffatomen bedeutet, und R, $R_1$ und $R_2$ die angegebene Bedeutung besitzen, ein Produkt der Formel:

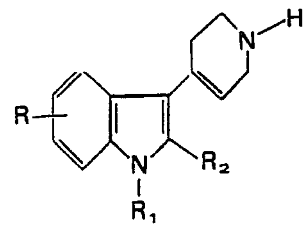
(II)

worin R, $R_1$ und $R_2$ die angegebene Bedeutung besitzen, mit einem Halogenid der allgemeinen Formel:

Hal−X'' (III')

worin Hal ein Chlor-, Brom- oder Jodatom bedeutet, und X'' einen Alkylrest mit 1 bis 6 Kohlenstoffatomen, einen Cycloalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen, einen Aralkylrest mit 7 bis 12 Kohlenstoffatomen oder einen Phenoxyalkylrest, worin der Alkylrest 1 bis 5 Kohlenstoffatome umfasst, bedeutet, umsetzt, um ein Produkt der Formel I zu erhalten, worin R, $R_1$, $R_2$ und X die angegebene Bedeutung besitzen, welches man isoliert und gewünschtenfalls mit einer Säure behandelt, um hieraus das Salz zu bilden.

2. Verfahren gemäss Anspruch 1 zur Herstellung von Derivaten der Formel I sowie von deren Additionssalzen mit Mineral- oder organischen Säuren, worin X einen Hydroxyalkylrest, worin der Alkylrest 2 bis 5 Kohlenstoffatome umfasst, bedeutet, und R, $R_1$ und $R_2$ die angegebene Bedeutung besitzen, dadurch gekennzeichnet, dass man ein Produkt der Formel:

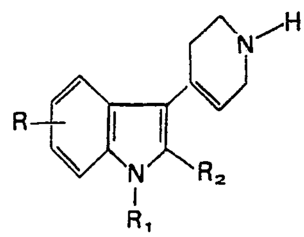
(II)

worin R, $R_1$ und $R_2$ die angegebene Bedeutung besitzen, mit einem Halogenid der Formel

Hal−X' (III)

worin Hal ein Chlor-, Brom- oder Jodatom bedeutet, und X' einen Tetrahydropyranyloxyalkylrest der Formel:

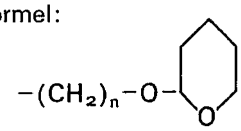
(IV)

bedeutet, worin n eine ganze Zahl entsprechend 2, 3, 4 oder 5 darstellt, umsetzt, um ein Derivat der Formel:

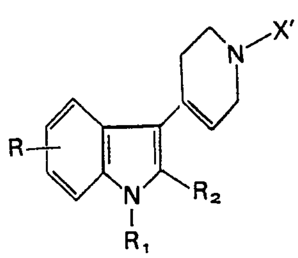
(V)

worin X', R, $R_1$ und $R_2$ die angegebene Bedeutung besitzen, zu erhalten, das Produkt der Formel V hydrolysiert, um ein Produkt der Formel I zu erhalten, worin X einen Hydroxyalkylrest darstellt, worin der Alkylrest 2 bis 5 Kohlenstoffatome umfasst, und R, $R_1$ und $R_2$ die angegebene Bedeutung besitzen, welches man isoliert und gewünschtenfalls mit einer Säure behandelt, um hieraus das Salz zu bilden.

3. Verfahren gemäss dem Anspruch 1 zur Herstellung von Derivaten der Formel I sowie von deren Additionssalzen mit Mineral- oder organischen Säuren, worin X einen Phenoxyalkylrest, in dem der Alkylrest 1 bis 5 Kohlenstoffatome umfasst, einen Alkylrest mit 1 bis 6 Kohlenstoffatomen, einen Cycloalkylrest mit 5 oder 6 Kohlenstoffatomen, einen Cycloalkylrest mit 4 bis 7 Kohlenstoffatomen, einen Alkenylrest mit 2 bis 5 Kohlenstoffatomen, einen Alkinylrest mit 3 bis 5 Kohlenstoffatomen oder einen Aralkylrest mit 7 bis 12 Kohlenstoffatomen bedeutet, und R, $R_1$ und $R_2$ die angegebene Bedeutung besitzen, dadurch gekennzeichnet, dass man ein Produkt der Formel:

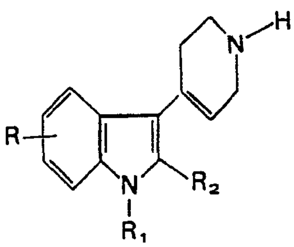

(II)

worin R, $R_1$ und $R_2$ die angegebene Bedeutung besitzen, mit einem Halogenid der allgemeinen Formel:

Hal−X" (III')

worin Hal ein Chlor-, Brom- oder Jodatom bedeutet und X" einen Alkylrest mit 1 bis 6 Kohlenstoffatomen, einen Cycloalkylrest mit 5 oder 6 Kohlenstoffatomen, einen Cycloalkylalkylrest mit 4 bis 7 Kohlenstoffatomen, einen Alkenylrest mit 2 bis 5 Kohlenstoffatomen, einen Alkinylrest mit 3 bis 5 Kohlenstoffatomen, einen Aralkylrest mit 7 bis 12 Kohlenstoffatomen oder einen Phenoxyalkylrest, in dem der Alkylrest 1 bis 5 Kohlenstoffatome umfasst, bedeutet, umsetzt, um ein Produkt der Formel I zu erhalten, worin R, $R_1$, $R_2$ und X die angegebene Bedeutung besitzen, welches man isoliert und gewünschtenfalls mit einer Säure behandelt, um hieraus das Salz zu bilden.

4. Verfahren gemäss einem der Ansprüche 1 oder 3, dadurch gekennzeichnet, dass man als Ausgangssubstanz ein Derivat der Formel (II), worin R einen 5-Nitrorest bedeutet, und $R_1$ und $R_2$ ein Wasserstoffatom bedeuten, und ein Halogenid der Formel III', worin X" einen Propylrest bedeutet, verwendet.

**Claims for the Contracting States:** BE, CH, DE, FR, GB, IT, LI, LU, NL, SE.

1. New N-substituted derivatives of tetrahydropyridinylindole and their salts of addition with mineral or organic acids, characterised in that they respond to the general formula:

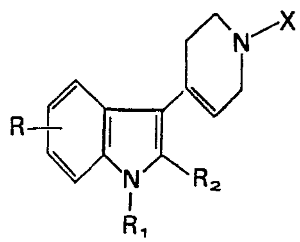

(I)

in which X represents an alkyl radical containing from 1 to 6 carbon atoms, cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms, an aralkyl radical containing from 7 to 12 carbon atoms, a hydroxy alkyl radical in which the alkyl radical contains from 2 to 5 carbon atoms or a phenoxyalkyl radical in which the alkyl radical contains from 1 to 5 carbon atoms, R represents a hydrogen or a halogen atom or an alkoxy radical containing from 1 to 3 carbon atoms, or a nitro, amino, trifluoromethyl or methylthio radical, $R_1$ and $R_2$ represent a hydrogen atom or an alkyl radical containing from 1 to 3 carbon atoms, it being understood that if R represents a hydrogen or a halogen atom or an alkoxy radical containing from 1 to 3 carbon atoms, X necessarily represents a hydroxyalkyl or phenoxyalkyl radical as defined above.

2. N-substituted derivative of tetrahydropyridinylindole and their salts of addition with mineral or organic acids, responding to formula (I) according to Claim 1 characterised in that X represents a hydroxyalkyl radical in which the alkyl radical contains from 2 to 5 carbon atoms and R, $R_1$ and $R_2$ have the already indicated significance.

3. N-substituted derivatives of tetrahydropyridinylindole and their salts of addition with mineral or organic acids, responding to the formula (I) according to Claim 1, characterised in that X represents a phenoxyalkyl radical in which the alkyl radical contains from 1 to 5 carbon atoms and R, $R_1$ and $R_2$ have the already indicated significance.

4. N-substituted derivatives of tetrahydropyridinylindole and their salts of addition with mineral or organic acids responding to formula (I) according to Claim 1, characterised in that X represents an alkyl radical containing from 1 to 6 carbon atoms, a cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms or an aralkyl radical containing from 7 to 12 carbon atoms and R, $R_1$ and $R_2$ have the already indicated significance.

5. The hydrochloride of 5-nitro 3-(1 propyl 1,2,3,6-tetrahydropyridin-4-yl) [1H]-indole.

6. Preparation process for the derivatives of formula (I) according to Claim 1 as well as their salts of addition with mineral or organic acids, in

which X represents a hydroxyalkyl radical in which the alkyl radical contains from 2 to 5 carbon atoms and R, $R_1$ and $R_2$ have the already indicated significance, characterised in that a product with formula:

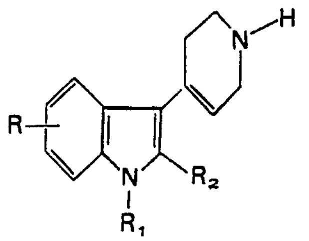

(II)

in which R, $R_1$ and $R_2$ have the already stated significance, is made to react with a halide with the formula:

Hal−X' (III)

in which Hal represents a chlorine, bromine or an iodine atom and X' represents a tetrahydropyranyloxyalkyl radical with the formula:

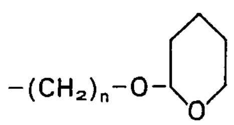

(IV)

in which n represents an integer 2, 3, 4 or 5, so as to obtain a derivative with the formula:

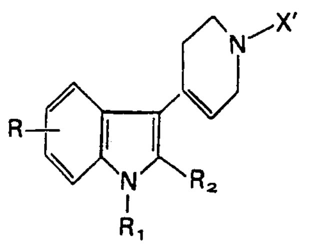

(V)

in which X', R, $R_1$ and $R_2$ have the already indicated significance, which product with the formula (V) is hydrolyzed so as to obtain a product with the formula (I) in which X represents a hydroxyalkyl radical in which the alkyl radical contains from 2 to 5 carbon atoms and R, $R_1$ and $R_2$ have the already indicated significance, which is isolated and, if necessary, treated with an acid so as to form its salt.

7. Preparation process for the derivatives with the formula (I) according to Claim 1 as well as their salts of addition with mineral or organic acids, in which X represents a phenoxyalkyl radical in which the alkyl radical contains from 1 to 5 carbon atoms, an alkyl radical containing from 1 to 6 carbon atoms, a cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms or an aralkyl radical containing from 7 to 12 carbon atoms and R, $R_1$ and $R_2$ have the already stated significance, characterised in that a product with the formula:

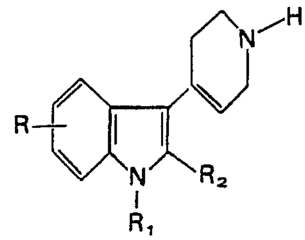

(II)

in which R, $R_1$ and $R_2$ have the already indicated significance is made to react with a halide with the general formula:

Hal−X'' (III')

in which Hal represents a chlorine, bromine or iodine atom and X'' represents an alkyl radical containing from 1 to 6 carbon atoms a cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms, an aralkyl radical containing from 7 to 12 carbon atoms or a phenoxyalkyl radical in which the alkyl radical contains from 1 to 5 carbon atoms, so as to obtain a product with the formula (I) in which R, $R_1$, $R_2$ and X have the already indicated significance, which is isolated and, if necessary, treated with an acid so as to form its salt.

8. Medicaments, characterised in that they are constituted by the new N-substituted derivatives of tetrahydropyridinylindole, as defined in formula (I) of Claim 1, as well as by their salts of addition with pharmaceutically acceptable acids.

9. Medicaments, characterised in that they are constituted by the new N-substituted derivatives of tetrahydropyridinylindole, as defined in one of the Claims 2, 3 or 4, as well as by their salts of addition with pharmaceutically acceptable acids.

10. Medicament, characterised in that it is constituted by the N-substituted derivative of tetrahydropyridinylindole, as defined in Claim 5.

11. Pharmaceutical compositions, characterised in that they contain as active principle, one at least of the medicaments, as defined at one of the Claims 8, 9 or 10.

**Claims for the Contracting State:** AT

1. Preparation process for new N-substituted derivatives of tetrahydropyridinylindole and their salts of addition with mineral or organic acids, characterised in that they respond to the general formula:

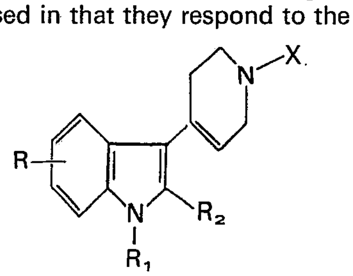

(I)

in which X represents an alkyl radical containing from 1 to 6 carbon atoms, cycloalkyl radical

containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms, an aralkyl radical containing from 7 to 12 carbon atoms, a hydroxyalkyl radical in which the alkyl radical contains from 2 to 5 carbon atoms or a phenoxyalkyl radical in which the alkyl radical contains from 1 to 5 carbon atoms, R represents a hydrogen or a halogen atom or an alkoxy radical containing from 1 to 3 carbon atoms, or a nitro, amino, trifluoromethyl or methylthio radical, $R_1$ and $R_2$ represent a hydrogen atom or an alkyl radical containing from 1 to 3 carbon atoms, it being understood that if R represents a hydrogen or a halogen atom or an alkoxy radical containing from 1 to 3 carbon atoms, X necessarily represents a hydroxyalkyl or phenoxyalkyl radical as defined above, characterised in that:

a) to prepare the derivative with the formula (I) as well as their salts of addition with mineral or organic acids, in which X represents a hydroxyalkyl radical in which the alkyl radical contains from 2 to 5 carbon atoms and R, $R_1$ and $R_2$ have the already indicated significance, a product with formula

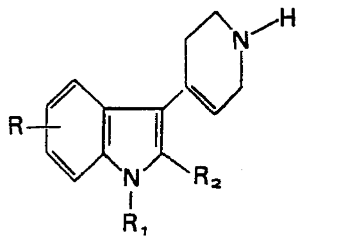

(II)

in which R, $R_1$ and $R_2$ have the already stated significance, is made to react with a halide with the formula:

Hal$-$X' (III)

in which Hal represents a chlorine, bromine or an iodine atom and X' represents a tetrahydropyranyloxyalkyl radical with the formula:

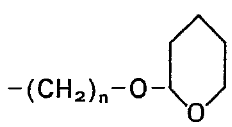

(IV)

in which n represents an integer 2, 3, 4 or 5, so as to obtain a derivative with the formula:

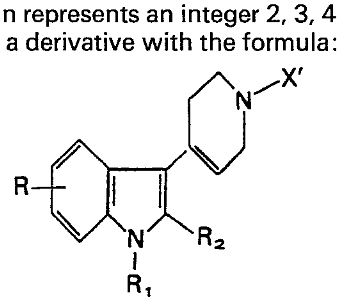

(V)

in which X', R, $R_1$ and $R_2$ have the already indicated significance, which product with the formula (V) is hydrolyzed so as to obtain a product with the formula (I) in which X represents a hydroxyalkyl radical in which the alkyl radical contains from 2 to 5 carbon atoms and R, $R_1$ and $R_2$ have the already indicated significance, which is isolated and, if necessary, treated with an acid so as to form its salt,

b) to prepare the derivatives with the formula (I), as well as their salts of addition with mineral or organic acids, in which X represents a phenoxyalkyl radical in which the alkyl radical contains from 1 to 5 carbon atoms, an alkyl radical containing from 1 to 6 carbon atoms, a cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms or an aralkyl radical containing from 7 to 12 carbon atoms and R, $R_1$ and $R_2$ have the already stated significance, characterised in that a product with the formula:

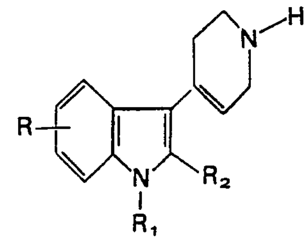

(II)

in which R, $R_1$ and $R_2$ have the already indicated significance is made to react with a halide with the general formula:

Hal$-$X" (III')

in which Hal represents a chlorine, bromine or iodine atom and X" represents an alkyl radical containing from 1 to 6 carbon atoms a cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms, an aralkyl radical containing from 7 to 12 carbon atoms or a phenoxyalkyl radical in which the alkyl radical contains from 1 to 5 carbon atoms, so as to obtain a product with the formula (I) in which R, $R_1$, $R_2$ and X have the already indicated significance, which is isolated and, if necessary, treated with an acid so as to form its salt.

2. Process according to Claim 1 for the preparation of the derivatives with the formula (I), as well as their salts of addition, with mineral or organic acids, in which X represents a hydroxyalkyl radical in which the alkyl radical contains from 2 to 5 carbon atoms and R, $R_1$ and $R_2$ have the already stated significance, characterised in that a product with the formula:

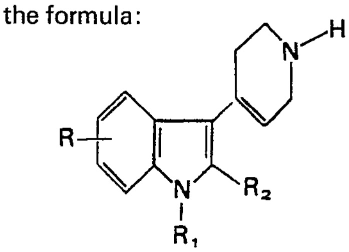

(II)

in which R, $R_1$ and $R_2$ have the already stated significance, is made to react with a halide with the formula:

Hal$-$X' (III)

in which Hal represents a chlorine, bromine or iodine atom and X′ represents a tetrahydropyranyl-oxyalkyl radical with the formula

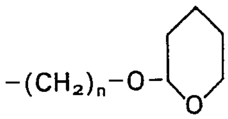

(IV)

in which n represents an integer 2, 3, 4 or 5, so as to obtain a derivative with the formula:

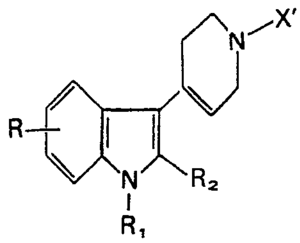

(V)

in which X′, R, $R_1$ and $R_2$ have the already indicated significance, which product with the formula (V) is hydrolysed so as to obtain a product with the formula (I) in which X represents a hydroxyalkyl radical, in which the alkyl radical contains from 2 to 5 carbon atoms and R, $R_1$ and $R_2$ have the already indicated significance, which is isolated and, if necessary, treated with an acid so as to form its salt.

3. Process according to Claim 1, for the preparation of the derivatives with formula (I) as well as their salts of addition with mineral or organic acids, in which X represents a phenoxyalkyl radical in which the alkyl radical contains from 1 to 5 carbon atoms, an alkyl radical containing from 1 to 6 carbon atoms, a cycloalkyl radical containing 5 or 6 carbon atoms, a cycloalkylalkyl radical containing from 4 to 7 carbon atoms, an alkenyl containing from 2 to 5 carbon atoms, an alkynyl radical containing from 3 to 5 carbon atoms, an alkynyl radical containing from 3 to 5 carbon atoms or an aralkyl radical containing from 7 to 12 carbon atoms and R, $R_1$, and $R_2$ have the already stated significance, characterised in that a product with the formula:

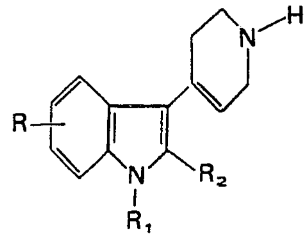

(II)

in which R, $R_1$ and $R_2$ have the already indicated significance is made to react with a halide with the general formula:

Hal−X″ (III′)

in which Hal represents a chlorine, bromine or iodine atom and X″ represents an alkyl radical containing from 1 to 6 carbon atoms, a cycloalkyl radical containing 5 or 6 carbon atoms, a cycloalkylalkyl radical containing from 4 to 7 carbon atoms, an alkenyl radical containing from 2 to 5 carbon atoms, an alkynyl radical containing from 3 to 5 carbon atoms, an aralkyl radical containing from 7 to 12 carbon atoms or a phenoxyalkyl radical in which the alkyl radical contains from 1 to 5 carbon atoms, so as to obtain a product with the formula (I) in which R, $R_1$ and $R_2$ and X have the already indicated significance, which is isolated, and if necessary, treated with an acid so as to form its salt.

4. Process according to Claim 1 or 3 characterised in that for starting a derivative with the formula (II) is utilised, in which R is a 5-nitro radical and $R_1$ and $R_2$ each represent a hydrogen atom and a halide with the formula (III′) in which X″ represents a propyl radical.